# EUROPEAN PATENT APPLICATION

(11) **EP 2 478 846 A2**
(43) Date of publication of application: **25.07.2012**
(21) Application number: 12152182.7
(22) Date of filing: 23.01.2012
(51) Int. Cl.: A61B 17/04

(54) **Wound closure device using t-shaped tag**

(30) Priority: 24.01.2011 US 201161435437 P; 13.01.2012 US 201213349978
(71) Applicant: Tyco Healthcare Group LP, Mansfield, MA 02048 (US)
(72) Inventor: Rockrohr, Brian, Waterbury, CT Connecticut 06705 (US); Fischvogt, Greg, Hamden, 06514 (US)
(74) Representative: Soames, Candida Jane

(57) **Abstract**

A suture assembly (12) is provided including a first suture (16) having a loop (20) formed at its distal end (22) and a second suture (18) having a cross member or T-tag (30) formed at its distal end (28). The T-tag (30) of the second suture (18) is engageable with the loop (20) of the first suture (16) when positioned within a body cavity in order to draw together a pair of tissue sections (A,D). An installation assembly (14) is also provided and generally includes first (32) and second (34) hypo-tubes for penetrating the first (A) and second (B) tissue sections and passing the first (16) and second (18) sutures through the first (A) and second (B) tissue sections. A plunger (52) is provided through one (34) of the hypo-tubes to eject the T-tag (30) out of the hypo-tube (34) and through the loop (20) of the first suture (16).

## Description

### CROSS-REFERENCE TO RELATED APPLICATIONS

This application claims priority to, and the benefit of, U.S. Provisional Patent Application Serial No. 61/435,437 filed January 24, 2011, the entire contents of which are incorporated by reference herein.

### BACKGROUND

### 1. Technical field

The present disclosure relates to wound closure devices and methods. More particularly, the present disclosure relates to a two-part suture assembly and an installation assembly for securing a pair of tissue sections together.

### 2. Background Of Related Art

During certain surgical procedures it is often necessary to secure cut or damaged edges of a pair of tissue sections together. This involves passing one or more sutures through first and second tissue sections and using the sutures or other alternative devices to draw the first and second tissue sections together. Thereafter, the free ends of the sutures are tied together to form a knot adjacent the outer surface of the tissue sections thereby securing the tissue sections together.

Manipulating a single length of suture through a pair of tissue sections, or manipulating a pair of sutures through a pair of tissue sections, is often a cumbersome procedure due to the flexible nature of the lengths of suture material utilized. Additionally, it is often difficult to secure free ends of a first and a second suture together within a body cavity.

Therefore, a need exists for a two-part suture assembly which can be easily connected together within a body cavity. Additionally, a need exists for an installation assembly capable of simply and precisely passing a pair of sutures through a pair of tissue sections.

### SUMMARY

There is disclosed a suture assembly for suturing together a pair of tissue sections. The suture assembly generally includes a first suture having a proximal end, a distal end and a loop of suture material provided at the distal end. A second suture is also provided and has a proximal end, a distal end and a cross member provided on the distal end of the second suture. The cross member is sized so as to be insertable through the loop of suture material. The cross member has a sharp, tissue penetrating tip.

In one embodiment, a trailing end of the loop is formed integrally with an initial end of the loop. In an alternative embodiment, the trailing end of the loop is attached to the initial end of the loop by forming a knot in the trailing end and securing the knot about the initial end of the loop. In a more specific embodiment, the knot formed in the trailing end is a slip knot.

In one embodiment, the cross member is formed integrally with the second suture while in an alternative embodiment the cross member includes a hole and the distal end of the second suture is affixed within the hole. In a particular embodiment, the distal end of the second suture is affixed within the hole in the cross member by gluing. In an alternative embodiment, the distal end of the second suture is affixed within the hole in the cross member by swaging.

In a particular embodiment, the cross member is formed of a polymer material similar, or identical, to the material of the second suture. Alternatively, the cross member is formed of any biocompatible material.

There is also disclosed a suture and installation assembly for suturing together a pair of tissue sections. The suture and installation assembly generally includes a first suture having a proximal end, a distal end and a loop of suture material provided at the distal end and a second suture having a proximal end, a distal end and a cross member provided on the distal end of the second suture. The cross member is insertable through the loop of suture material. The suture and installation assembly additionally includes a hollow installation tube or hypo-tube for receiving and passing at least one of the first and second sutures through tissue.

The hypo-tube has a sharp, tissue penetrating distal tip and the tissue penetrating distal tip has an angled face.

A plunger is provided and is insertable through the hollow hypo-tube. The plunger includes a distal end engageable with a proximal end of the cross member to eject the cross member out of the hypo-tube. The plunger additionally includes a shaft having a longitudinal groove for receipt of the second suture to prevent the second suture from binding within the hypo-tube.

In another aspect of the present disclosure, a suture and instrument assembly for suturing tissue includes a suture having a proximal end, a distal end, and an anchor member secured to the distal end of the suture. The suture and instrument assembly also includes a hypo-tube defining a longitudinal axis and having a proximal portion, a distal portion, and a bore therethrough. A plunger is slidably disposed in the bore. A housing is coupled to the proximal portion of the hypo-tube, and a carrier is disposed within the bore in the distal portion of the hypo-tube. The anchor member is slidably disposed on the carrier.

In a further aspect of the present disclosure the carrier includes a sloped surface. An anchor member is slidably disposed on the sloped surface. The sloped surface is disposed at an angle between 0 and 180 degrees with respect to the longitudinal axis. In another aspect of the present disclosure, the a needle extends distally away from the housing. The needle may include a tissue-penetrating surface.

In another aspect of the present disclosure the housing includes a handle, the handle operably coupled with the plunger. A spring assembly may be operatively coupled to the handle assembly.

There is further disclosed a method of suturing a first and a second tissue section. The method includes providing a first suture having a proximal end, a distal end and a loop of suture material provided at the distal end and a second suture having a proximal end, a distal end and a cross member provided on the distal end of the second suture. The cross member is insertable through the loop of suture material. The loop of the first suture is passed through a first tissue section and the cross member is passed through a second tissue section. The cross member is then passed through the loop in the first suture such that the cross member engages the loop. Thereafter, at least one of the proximal ends of the first and second sutures is tensioned to draw the first and second tissue sections together.

The method further includes the step of tying the proximal ends of the first and second sutures together to secure the first and second tissues sections.

In a specific embodiment of the disclosed method, the loop of the first suture is passed through the first tissue section by inserting the loop in a hollow hypo-tube and inserting the hypo-tube through the first tissue section. The cross member is passed through the second tissue section by inserting the cross member in a hollow hypo-tube and inserting the hollow hypo-tube through the second tissue section. The cross member is passed through the second tissue section

by engaging the cross member with a plunger and manipulating the plunger through the hypo-tube to eject the cross member out of the hollow installation tube.

### DESCRIPTION OF THE DRAWINGS

Embodiments of the presently disclosed wound closure device are disclosed herein with reference to the drawings, wherein:
FIG. 1 is a perspective view of a wound closure device using a T-tag;
FIG. 2 is perspective view, with parts separated, of the wound closure device of FIG. 1 and incorporating a plunger;
FIG. 3 is an enlarged view of an area of detail of FIG. 2;
FIG. 4 is a first embodiment of a suture loop;
FIG. 5 is a second embodiment of the suture loop;
FIG. 6 is an enlarged view of an area of detail of FIG. 2;
FIG. 7 is a first embodiment of a suture T-tag assembly;
FIG. 8 is a second embodiment of the suture T-tag assembly;
FIG. 9 is a cross-sectional view of the plunger positioned within a hypo-tube of the wound closure device;
FIG. 10 is a perspective view, partially shown in section, illustrating the initial insertion of the wound closure device into tissues;
FIG. 11 is an enlarged view, partially shown in section, of the area of detail of FIG 10;
FIG. 12 is a perspective view, partially shown in section, illustrating the deployment of the T-tag assembly through the suture loop;
FIG. 13 is a view similar to FIG. 12 with hypo-tubes of the wound closure device removed;
FIG. 14 is a view similar to FIG. 13 after initial tensioning of the sutures to approximate the tissues;
FIG. 15 is a view similar to FIG. 14 with the suture loop and T-tag secured against undersurfaces of the tissues and tag ends of the sutures knotted against upper surfaces of the tissues to secure the tissues together;
FIG. 16 is a perspective view of an alternative embodiment of a hypo-tube according to the present disclosure;
FIG. 17 is a cut-away view taken along section line 17-17 of FIG. 16;
FIG. 18 is an enlarged cross-sectional view of a distal portion of the hypo-tube taken along section line 18-18 of FIG. 17
FIG. 19 is the cut-away view of FIG. 17, with the hypo-tube being actuated;
FIG. 20 is the enlarged cross-sectional view of FIG. 18, with the hypo-tube being actuated; and
FIG. 21 is a front view of the hypo-tube of FIG. 16 cooperating with another hypo-tube to effect wound closure.

### DETAILED DESCRIPTION OF EMBODIMENTS

Embodiments of the presently disclosed wound closure device will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

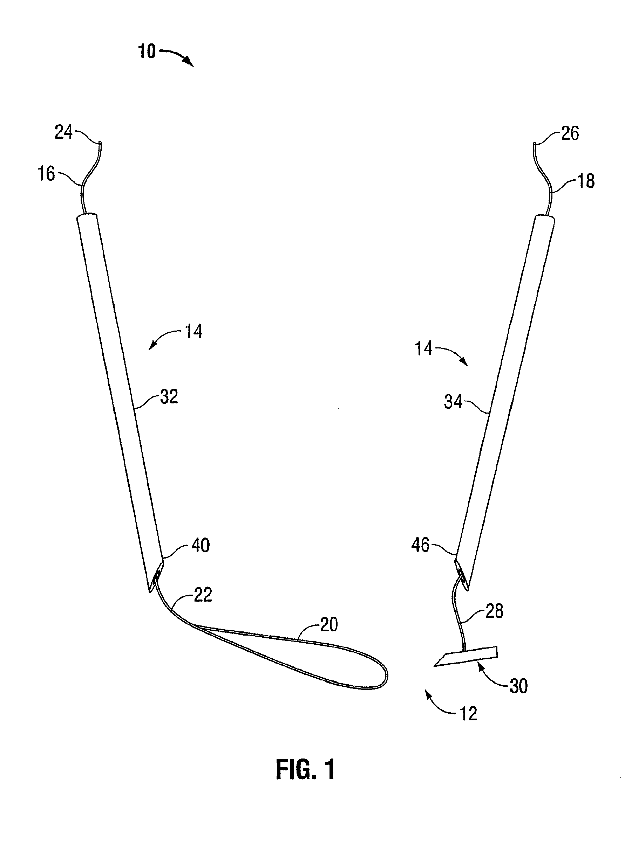

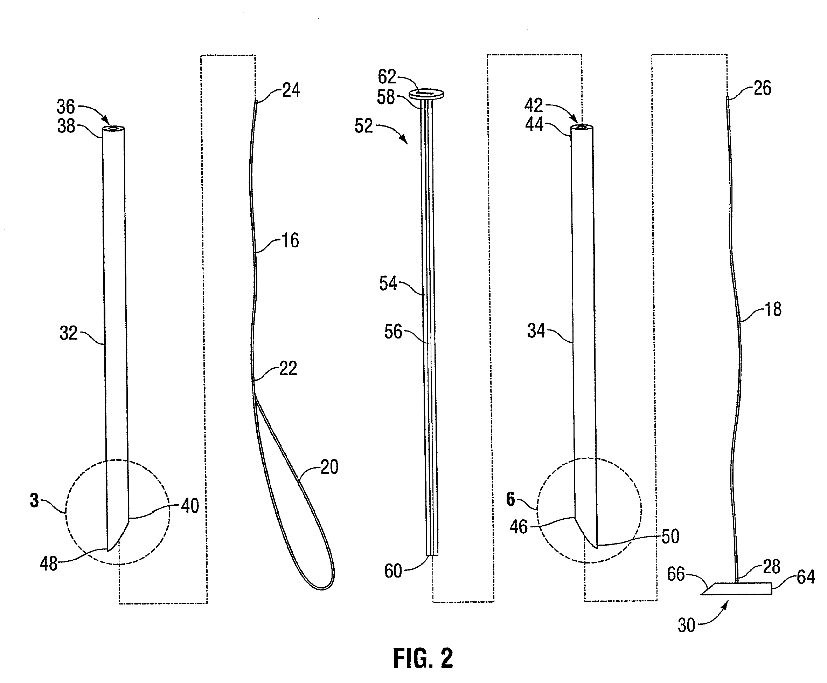

Referring to FIGS. 1 and 2, there is disclosed a wound closure device 10 for use in suturing a pair of wound edges or tissue sections together. Wound closure device 10 generally includes a suture assembly 12 and an installation assembly 14 for inserting suture assembly 12 through the tissue sections. Suture assembly 12 includes a first suture 16 and a second suture 18. First suture 16 has a loop 20 formed on a distal end 22 thereof for engagement with second suture 18. First suture 16 has a proximal free or tag end 24 which is provided to be tied off to a free or tag end 26 of second suture 18 to secure the tissue sections. In order to connect a distal end 28 of second suture 18 to loop 20 formed on distal end 22 of first suture 16, second suture 18 includes a cross member or T-tag 30 located at distal end 28 and positionable within loop 20 of first suture 16 as described in more detail hereinbelow.

First and second sutures 16 and 18 are formed of relatively flexible materials of the type typically used in the formation of sutures such as, for example, polymer materials, etc. Loop 20 may be treated in a manner which renders loop 20 slightly stiffer than the remaining length of suture 16 so as to allow loop 20 to maintain its open shape. T-tag 30 provided on distal end 28 of suture 18 is formed of a relatively rigid material such as, for example, stainless steel, polymer material, etc.

In order to insert first and second sutures 16 and 18 through tissues, installation assembly 14 includes a pair of hypo-tubes such as, for example, first and second hypo-tubes 32 and 34, which are provided to penetrate the tissues and allow first and second sutures 16 and 18 to pass through the tissues. First hypo-tube 32 defines a through bore 36 extending therethrough from an open proximal end 38 to an open distal end 40. Likewise, second hypo-tube 34 defines a through bore 42 extending between an open proximal end 44 to an open distal end 46.

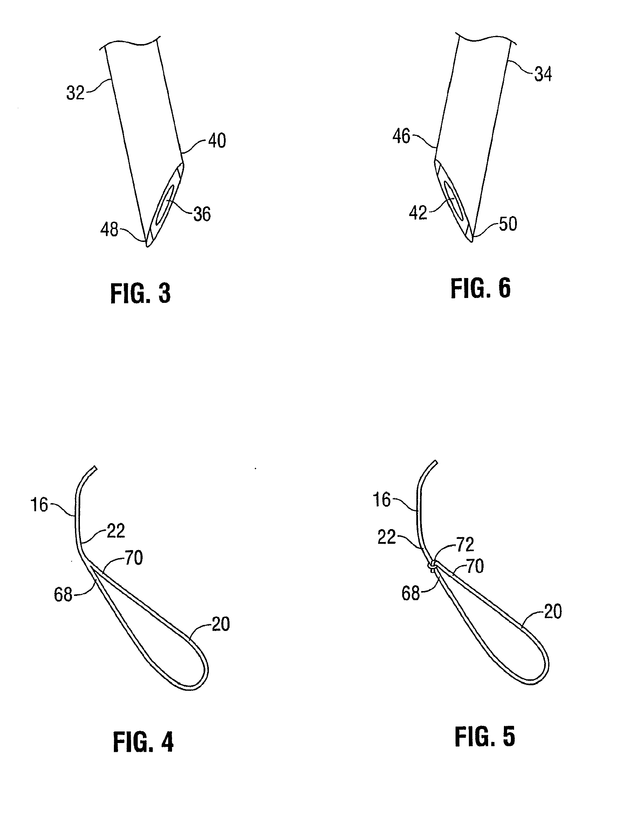

Referring for the moment to FIG. 3, in order to assist in penetrating through tissue, distal end 40 of first hypo-tube 32 includes a sharp, angled tissue penetrating tip 48. Similarly, with reference to FIG. 6, distal end 46 of second hypo-tube 34 also includes a sharp, angled tissue penetrating tip 50.

Referring back to FIG. 2, in order to facilitate ejecting T-tag 30 out of through bore 42 in second hypo-tube 34, installation assembly 14 additionally includes a plunger 52 having a grooved shaft 54 including a longitudinal groove 56. Groove 56 extends from a proximal end 58 of shaft 54 to a distal end 60 of shaft 54. An end cap 62 is provided at proximal end 58 to facilitate manipulating plunger 52 through second hypo-tube 34. Distal end 60 of shaft 54 is flat and is configured to engage a relatively flat proximal end 64 of T-tag 30 to drive or eject T-tag 30 out of second hypo-tube 34. T-tag 30 additionally includes an angled face or tissue penetrating distal tip 66.

Referring now to FIG. 4, and as noted hereinabove, first suture 16 includes loop 20 formed at distal end 22 of first suture 16. Loop 20 includes an initial end 68 and a trailing end 70. In this embodiment, initial end 68 and trailing end 70 are formed integrally with distal end 22 of first suture 16.

As best shown in FIG. 5, in an alternative embodiment, initial end 68 is formed integrally with distal end 22 of first suture 16. Trailing end 70 is attached to distal end 22 of first suture 16 by forming a knot 72 about trailing end 68. In this particular embodiment, knot 72 is a slip knot which can slide down trailing end 68 in order to cinch loop 20 down about T-tag 30 during suturing of tissue.

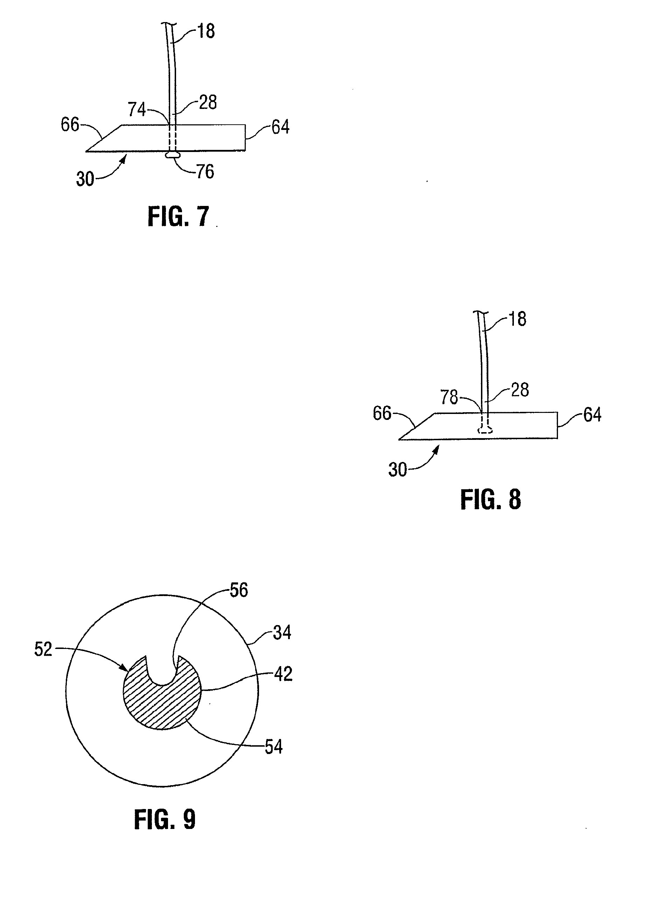

Referring now to FIG. 7, as noted hereinabove, T-tag 30 includes flat proximal end 64 and tissue penetrating distal tip 66. In order to attach T-tag 30 to second suture 18, T-tag 30 includes a central through bore 74 for receipt of distal end 28 of second suture 18. In this embodiment, distal end 28 extends completely through through bore 74 and is secured to T-tag 30 by forming a knot in distal end 28 or by melting distal end 28 to form a bulge 76 in distal end 28 thereby securing second suture 18 to T-tag 30.

In an alternative embodiment, and with reference to FIG. 8, T-tag 30 includes a partial bore 78 for receipt of distal end 28 of second suture 18. In this embodiment, distal end 28 is glued, welded, swaged, etc. to secure distal end 28 within partial bore 78.

Referring now for the moment to FIG. 9, and as noted hereinabove, plunger 52 is provided within through bore 42 of second hypo-tube 34. Shaft 54 of plunger 52 is provided with groove 56 to receive second suture 18 and prevent second suture 18 from binding between shaft 54 and through bore 42.

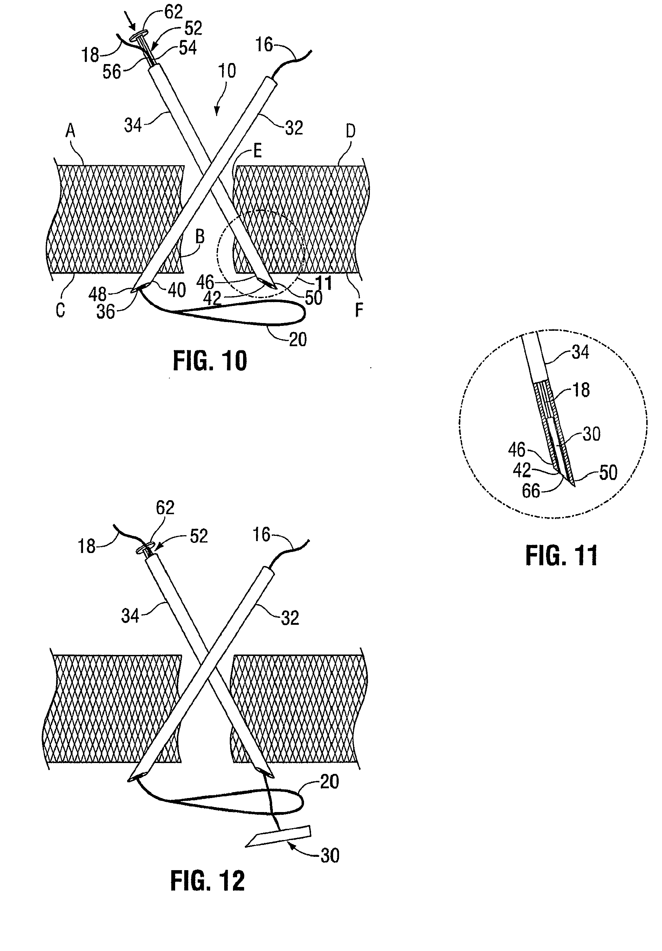

Referring now to FIGS. 10-15, and initially with regard to FIG. 10, the use of wound closure device 10 to suture a first tissue section A having an edge B and an inner surface C to a second tissue section D having an edge E and an inner surface F will now be described. Initially, first suture 16, including loop 20, is positioned within the first hypo-tube 32 and first hypo-tube 32 is manipulated such that tissue penetrating tip 48 penetrates first edge B and exits through first inner surface C. First suture 16 is manipulated distally through first hypo-tube 32 such that loop 20 exits first through bore 36. At this point, first hypo-tube 32 may be removed and utilized in conjunction with plunger 52 to insert second suture 18 and T-tag 30 through second tissue section D.

Alternatively, first hypo-tube 32 is left in position extending through first tissue section A. Second hypo-tube 34 and second suture 18 are assembled such that T-tag 30 is positioned within second through bore 42 and adjacent open distal end 40 of second hypo-tube 34 (see FIG. 11). Plunger 52 is positioned within second through bore 42 such that second suture 18 lies within groove 56 in shaft 54 of plunger 52. In use, cap 62 of plunger 52 is driven distally relative to second hypo-tube 34 to drive shaft 54 distally within second through bore 42.

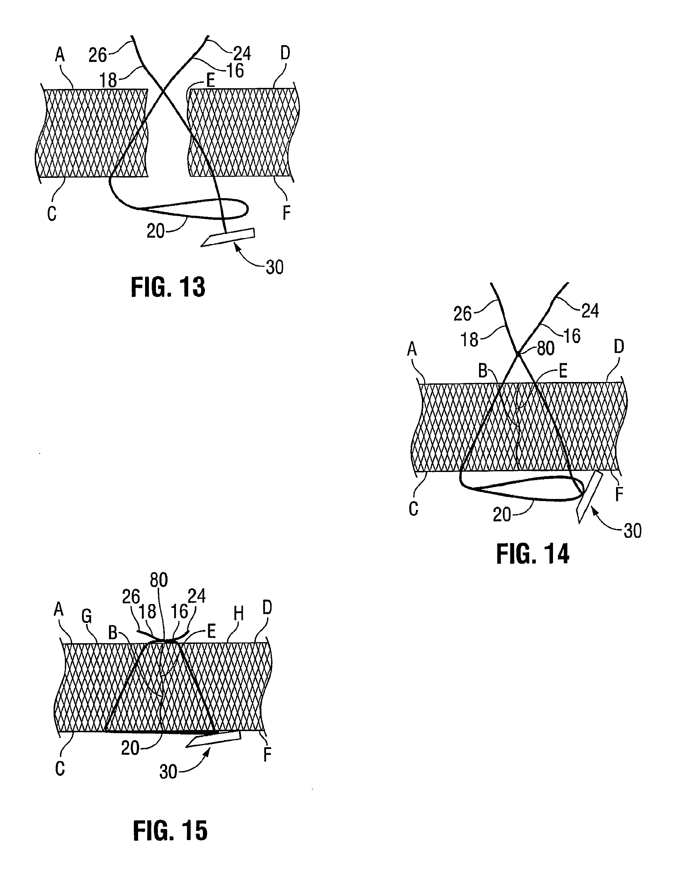

Referring now to FIG. 12, open distal end of 40 of second hypo-tube 34 is positioned adjacent loop 20 of first suture 16. As plunger 52 continues to be driven distally through second hypo-tube 34, T-tag 30 is ejected out of open distal end 42 such that T-tag 30 passes through loop 20. At this point, first and second hypo-tubes 32 and 34 are removed from the respective tissue sections A and B, leaving first and second sutures 16 and 18 extending through first and second tissue sections A and D as shown in FIG. 13.

With reference to FIGS. 13 and 14, proximal or tag ends 24 and 26 of first and second sutures 16 and 18, respectively, are tensioned to cause T-tag 30 into engagement with loop 20 and draw first and second edges B and E of first and second tissue sections A and D together. Thereafter, with reference to FIGS. 14 and 15, a suture knot 80 is tied between first suture 16 and second suture 18 and is advanced distally down against a first outer surface G of first tissue section A and a second outer surface H of second tissue section D to thereby secure first and second tissue sections A and D together. As shown, loop 20 and T-tag 30 are secured against undersurfaces C and F of first and second tissue sections A and D.

In this manner, wound closure device 10, including suture assembly 12 and installation assembly 14, provides a safe and simple method of suturing together a pair of tissue sections.

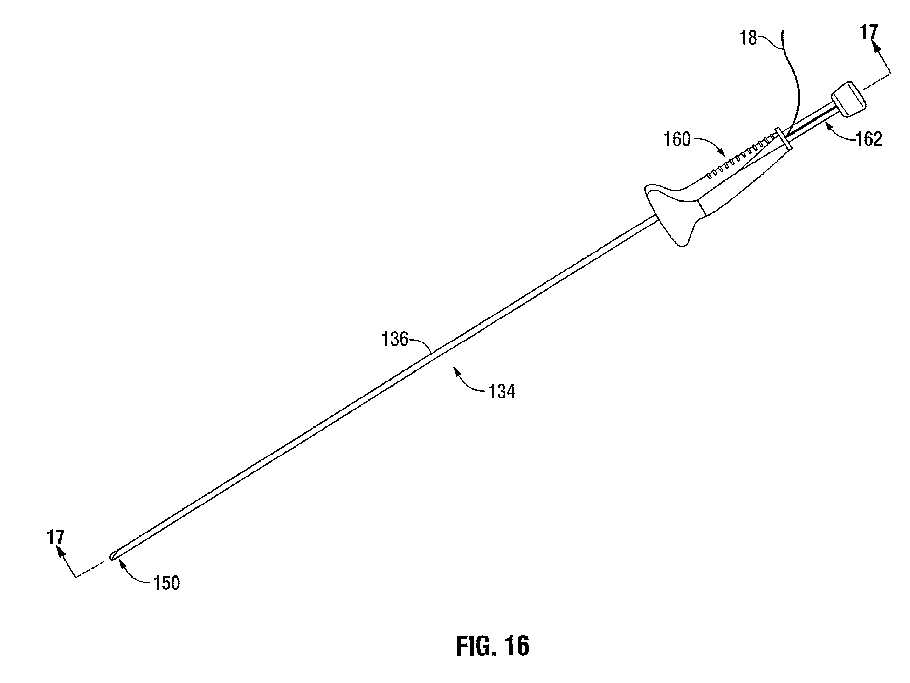

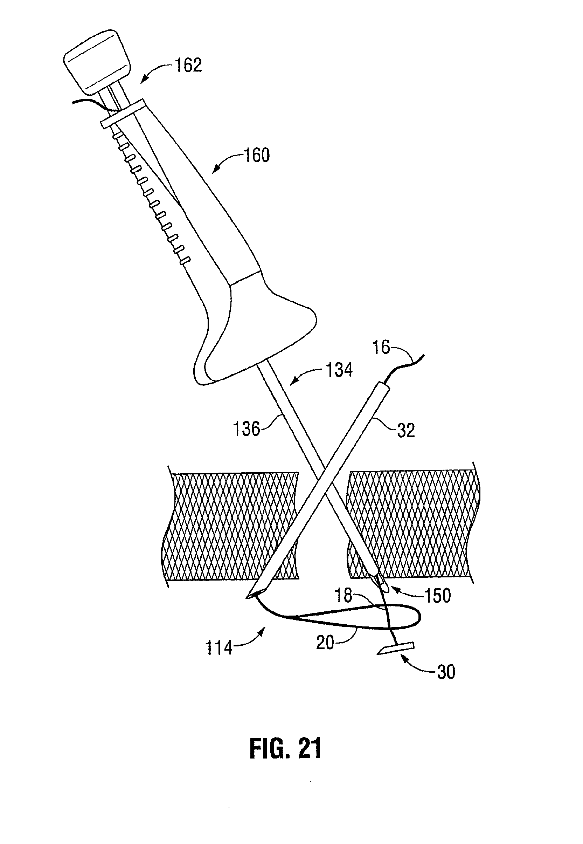

Turning to FIG. 16, a perspective view of a portion of an installation assembly 114 (FIG. 21) includes hypo-tube 134. Hypo-tube 134 may be used in conjunction with hypo-tube 32 (FIG. 1) and suture 16 (FIG. 1) described above, or with another desirable instrument for introducing a suture 16 with a loop 20 (FIG. 1) through tissue.

Hypo-tube 134 defines a longitudinal axis A, and includes a housing 160 and a handle 162. Housing 160 is coupled to a proximal portion of hypo-tube 134, and handle 162 slidably disposed within the housing 160. An elongate tube or needle 136 extends distally away from the housing 160. Needle 136 includes a distal portion 150.

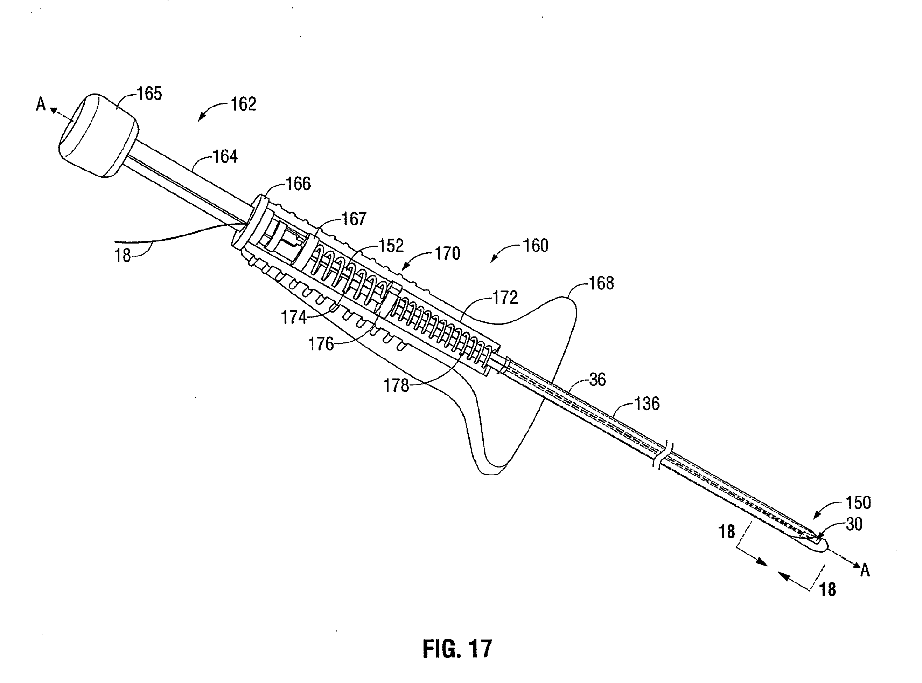

Turning to FIG. 17, a cut-away view of the hypo-tube 134 is shown. An internal bore 36 or lumen is defined within needle 136, and opens to a lateral surface of the distal portion 150 of needle 136. Suture 18, as shown, is disposed through hypo-tube 134 and may exit an aperture of housing 160, or may exit another portion of hypo-tube 134. T-tag 30 is disposed within the bore 36 at the distal portion 150 of needle 136.

Housing 160 tapers proximally, and has a distal region defined by a flange 168. Housing 160 additionally defines an internal channel 172 in which a proximal end of needle 136 is disposed. A plunger 152 is slidably disposed within bore 36 of needle 136, and extends proximally away from the proximal end of needle 136 to contact a pusher plate 167, as will be described further below.

A spring assembly 170 is disposed within channel 172, and is concentrically disposed about plunger 152 and needle 136. Accordingly, plunger 152 and needle 136 may translate through spring assembly 170 substantially uninhibited. Spring assembly 170 includes a proximal spring 174 and a distal spring 178. Proximal and distal springs 174, 178 may have different dimensions or spring constants. Proximal spring 174 and distal spring 178 are separated by a slide 176 that is slidably disposed within channel 172. Slide 176 includes an opening through which needle 136 is disposed. Slide 176 may slidably engage needle 136, or may be free from contact with needle 136. A proximal end of proximal spring 174 abuts pusher plate 167, as will be discussed further below, and a distal end of proximal spring 174 abuts slide 176. Similarly, a proximal end of distal spring 178 abuts slide 176, and a distal end of distal spring 178 abuts a ledge formed at the distal end of channel 172. Accordingly, proximal and distal springs 174, 178 may be compressed within channel 172 upon a distally applied force, as will be discussed further below

A handle 162 of the hypo-tube 134 is operably coupled with plunger 152 and includes a proximal knob 165 and a shaft 164 extending distally therefrom. Shaft 164 is slidably disposed through a cap 166. Cap 166 is fit into a proximal end of the housing 160 such that axial forcing in the course of operation will not disengage cap 166 from housing 160. Accordingly, cap 166 may be press-fit, adhered, or otherwise secured to housing 160. A pusher plate 167 is disposed at a distal end of the shaft 164 of handle 162, and is slidably disposed within channel 172. Accordingly, pusher plate 167 is operatively connected to shaft 164 of handle 162, and contacts a proximal end of proximal spring 174.

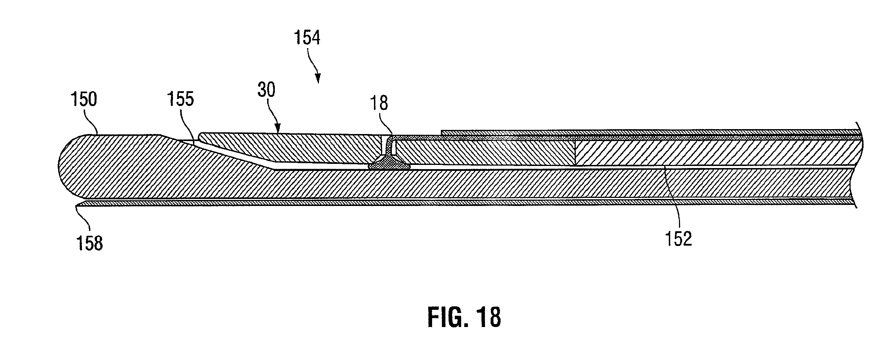

Turning to FIG. 18, the distal portion 150 of needle 136 is shown in detail. Distal portion 150 of needle 136 may include a distally-extending tissue-penetrating surface 158 for insertion into tissue, as will be described further below. A carrier 154 is disposed within the distal portion 150 of needle 136, and includes an inwardly-sloping surface 155 that extends into bore 36 of needle 136 (FIG. 17). Inwardly-sloping surface 155 may be disposed at an angle between 0 and 180 degrees with respect to the longitudinal axis A (FIG. 17). The T-tag 30 is slidably disposed on the inwardly-sloping surface 155, such that the inwardly-sloping surface 155 provides an exit path for the T-tag 30 from the bore 36. The distal end 28 (FIG. 1) of suture 18 is secured to T-tag 30 or another suitable anchor member, and extends proximally through bore 36 of needle 136 (FIG. 17).

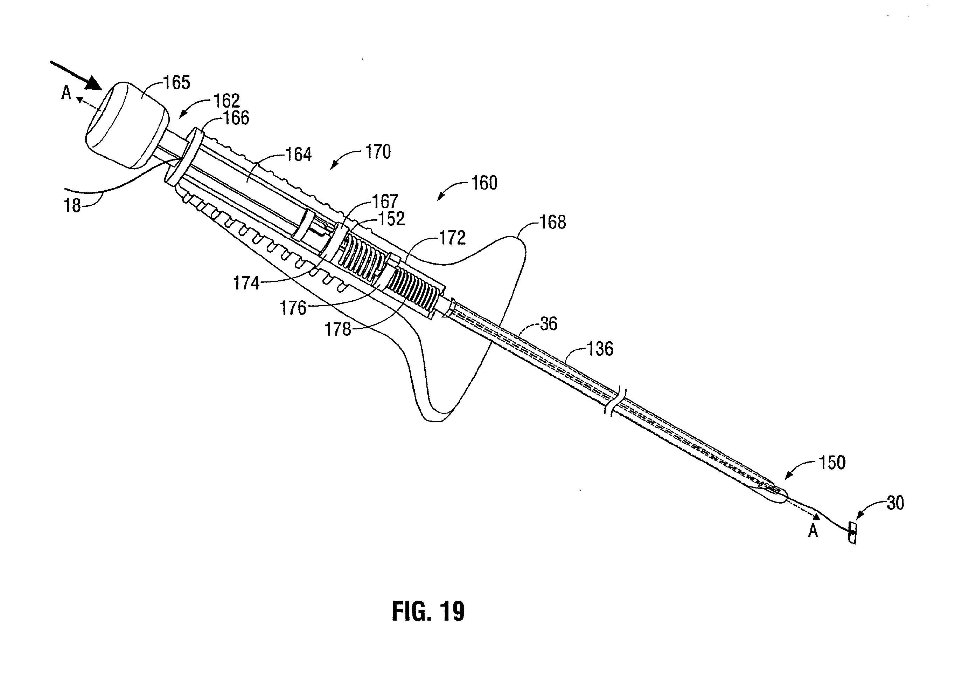

Turning to FIG. 19, actuation of the hypo-tube 134 is shown. An operator or actuation mechanism grasps knob 165 and depresses shaft 164 of handle 162 distally toward housing 160. Flange 168 of housing 160 provides an additional surface for engagement by an operator or actuation mechanism. As the shaft 164 travels distally, pusher plate 167 causes the compression of proximal and distal springs 174, 178 within channel 172. Slide 176 will translate distally through channel 172 with the compression of proximal and distal springs 174, 178. The resistance encountered by the compression of proximal and distal springs 174, 178 provides a counterforce to the distal force exerted on the handle 162. In this manner, inadvertent overforcing of the handle 162 is minimized. Further, a return force generated by the compressed proximal and distal springs 174, 178 will cause the handle 162 to return to its resting position after the distal force exerted by an operator or actuation mechanism is discontinued.

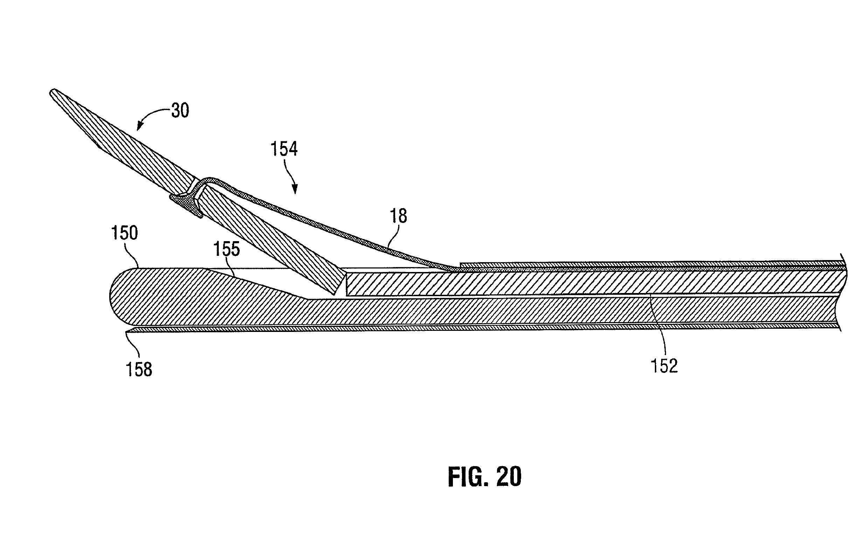

As the pusher plate 167 travels distally through channel 172, plunger 152 is advanced distally through needle 136. Referring to FIG. 20, a distal end of pusher 152 contacts T-tag 30 and, upon further movement, causes the T-tag 30 to eject from the bore 36 of needle 136 as shown.

Turning to FIG. 21, the hypo-tube 134 is shown disposed through a section of tissue, with hypo-tube 32 disposed through an opposing section of tissue. It will be understood by those skilled in the art that hypo-tube 32 may be configured in a similar manner to hypo-tube 134 for the purpose of introducing suture 16 through a section of tissue. Intercooperation of the T- tag 30 with loop 20 of suture 16 proceeds in a substantially similar manner as described above with respect to the previous embodiments. In particular, T-tag 30 may be inserted through loop 20 of suture 16. Hypo-tubes 134 and 32 may then be withdrawn from the respective sections of tissue, and the sutures 16, 18 may be drawn together and secured at a tissue surface in the manner described above to effect closure of the sections of tissue.

Specifically, with reference to FIGS. 13 and 14, and as described above, proximal or tag ends 24 and 26 of first and second sutures 16 and 18, respectively, are tensioned to cause T-tag 30 into engagement with loop 20 and draw first and second edges B and E of first and second tissue sections A and D together. Thereafter, with reference to FIGS. 14 and 15, a suture knot 80 is tied between first suture 16 and second suture 18 and is advanced distally down against a first outer surface G of first tissue section A and a second outer surface H of second tissue section D to thereby secure first and second tissue sections A and D together. As shown, loop 20 and T-tag 30 are secured against undersurfaces C and F of first and second tissue sections A and D.

It will be understood that various modifications may be made to the embodiments disclosed herein. For example, as noted hereinabove, the disclosed method may be performed utilizing either one or two hypo-tubes. Further, alternative anchoring structure may be provided in place of the disclosed T-tag, such as, for example, a disk, ball, or other enlarged structure may be provided on the distal end of the second suture for engagement with the loop of the first suture. Additionally, as noted hereinabove, the loop of the first suture may be formed with a slip knot which can be cinched down against the enlarged structure on the distal end of the second suture. Therefore, the above description should not be construed as limiting, but merely as exemplifications of particular embodiments. Those skilled in the art will envision other modifications within the scope and spirit of the claims appended hereto.

### The invention may be described by reference to the following numbered paragraphs:-

1. A suture and instrument assembly for suturing tissue, comprising:
   a suture having a proximal end, a distal end, and an anchor member secured to the distal end of the suture;
   a hypo-tube having a proximal portion, a distal portion, and a bore therethrough;
   a housing coupled to the proximal portion of the hypo-tube;
   a carrier disposed in a distal portion of the bore, the anchor member being slidably disposed on the carrier;
   a plunger slidably disposed in the bore, wherein the housing includes a handle operably coupled with the plunger; and
   a spring assembly operatively coupled to the handle assembly.
2. The suture and instrument assembly as recited in paragraph 1, wherein the carrier includes a sloped surface and the anchor member slidably disposed on the sloped surface.
3. The suture and instrument assembly as recited in paragraph 2, wherein the sloped surface is disposed at an angle between 0 and 180 degrees with respect to the longitudinal axis.
4. The suture and instrument assembly as recited in paragraph 1, wherein the hypo-tube includes a needle extending distally away from the housing.
5. The suture and instrument assembly as recited in paragraph 1, wherein the needle includes a tissue-penetrating surface.

## Claims

1. A suture and instrument assembly for suturing tissue, comprising:
a suture having a proximal end, a distal end, and an anchor member secured to the distal end of the suture;
a hypo-tube having a proximal portion, a distal portion, and a bore therethrough;
a housing coupled to the proximal portion of the hypo-tube;
a carrier disposed in a distal portion of the bore, the anchor member being slidably disposed on the carrier;
a plunger slidably disposed in the bore, wherein the housing includes a handle operably coupled with the plunger; and
a spring assembly operatively coupled to the handle assembly.

2. The suture and instrument assembly as recited in claim 1, wherein the carrier includes a sloped surface and the anchor member slidably disposed on the sloped surface.

3. The suture and instrument assembly as recited in claim 2, wherein the sloped surface is disposed at an angle between 0 and 180 degrees with respect to the longitudinal axis.

4. The suture and instrument assembly as recited in any preceding claim, wherein the hypo-tube includes a needle extending distally away from the housing.

5. The suture and instrument assembly as recited in claim 4, wherein the needle includes a tissue-penetrating surface.
